# EUROPEAN PATENT APPLICATION

(11) **EP 4 258 283 A1**
(43) Date of publication of application: **11.10.2023**
(21) Application number: 22736871.9
(22) Date of filing: 07.01.2022
(51) Int. Cl.: G16H 50/70, G16H 50/50, G16B 40/20, G16H 30/20, G16H 30/40, G16H 70/60, G16B 45/00, G06N 20/00, G06N 3/08, G06N 3/04

(54) **METHOD FOR TRAINING ARTIFICIAL NEURAL NETWORK PROVIDING DETERMINATION RESULT OF PATHOLOGICAL SPECIMEN, AND COMPUTING SYSTEM FOR PERFORMING SAME**

(30) Priority: 07.01.2021 KR 20210001912
(71) Applicant: Deep Bio Inc., Seoul 08380 (KR)
(72) Inventor: KWAK, Tae Yeong, Seoul 05119 (KR); PAIK, In Young, Seoul 03680 (KR); KIM, Sun Woo, Seongnam-si, Gyeonggi-do 13599 (KR)
(74) Representative: Flügel Preissner Schober Seidel
(86) International application number: PCT/KR2022/000269
(87) International publication number: WO 2022/149894

(57) **Abstract**

Disclosed are a method and a computing system for performing same, wherein an artificial neural network is trained using pathology slides, obtained by staining serial sections of a single pathological specimen with a variety of different staining reagents, such that a disease can be determined with a high degree of accuracy. One aspect of the present invention provides a method for training an artificial neural network, the method comprising: generating a training data set including M pieces of individual training data; and training the artificial neural network on the basis of the training data set. The step of generating the training data set including M pieces of individual training data includes the step of generating, for all m where 1<=m<=M, m-th training data to be included in the training data set. The step of generating the m-th training data includes the steps of: acquiring first to Nth pathology slide images, the first to Nth pathology slide images being pathology slide images obtained by staining serial sections of a single pathological specimen with different staining reagents; and generating the m-th training data on the basis of the first to Nth pathology slide images.

## Description

### TECHNICAL FIELD

The present invention relates to a method for training an artificial neural network that provides a result of determination on a pathological specimen, and a computing system for performing the same, and more specifically, to a method of determining a disease with high accuracy by training the artificial neural network using pathology slides obtained by staining serial sections of a single pathological specimen with a variety of different staining reagents, and a computing system for performing same.

### BACKGROUND ART

Neighboring cells interacting with tumor cells around a tumor and affecting growth of the tumor are called as a tumor microenvironment. Researches on the tumor microenvironment are very important in diagnosing and prognosing a current state of cancer, and in identifying responsiveness to a specific treatment method and developing new treatment methods.

Previously, immunohistochemistry (IHC) staining reagents are used targeting specific immune cells or proteins expected to be present in the tumor microenvironment in order to analyze the tumor microenvironment. That is, a process of staining a pathological specimen with an IHC staining reagent for a specific target and visually reading the staining result through an optical microscope is performed by a pathologist to grasp a positional relation of the targets and quantify an amount thereof. At this point, since it should be determined by comprehensively seeing various immune cells or proteins, a number of slides configured of serial sections of a single specimen are manufactured, and each of the slides is stained with H&E and various target IHC staining reagents, and thereafter, a pathologist reads and integrates results of the reading. The positional relation of various elements constituting the tumor microenvironment is important, and there is a limit in such an analysis type, and although a multiplex IHC staining method of staining various targets together has been introduced to overcome the limit, it is expensive and not generalized.

Meanwhile, techniques for diagnosing the current state of cancer, prognosing through classification of severity, and measuring results of staining with a specific IHC through analysis of pathology slide images using deep learning, particularly, convolutional neural network (CNN), are developed and commercialized. Since currently developed techniques are targeting single stained pathology slide images and analysis results are provided in the form of a specific classification or number, it is not easy to grasp the tumor microenvironment, together with the positional relations, by simply combining the analysis results. For example, according to an existing patent (JP6650453B2), although slide images configured of serial sections are used for prognosis of cancer, a prognosis is determined by integrating scores resulting from separate analysis of the slide images, and a comprehensive analysis of the positional relations of all slide images is not performed.

Accordingly, it is required to provide a technical idea capable of comprehensively analyzing multi-type-stained slides configured of serial sections of a single specimen, including positional relations, so that the tumor microenvironment can be analyzed without using a multiplex IHC method.

### * Prior art documents

### - Patent documents

Japanese Registered Patent Publication JP6650453 B2

### DISCLOSURE OF INVENTION

### TECHNICAL PROBLEM

Therefore, the present invention has been made in view of the above problems, and it is an object of the present invention to provide a method and system that can diagnose and prognose the current state of cancer, and grasp responsiveness to a specific treatment method with high accuracy by training an artificial neural network using pathology slides obtained by staining serial sections of a single pathological specimen with H&E or a variety of target IHC staining reagents so that the trained artificial neural network may comprehensively analyze the tumor microenvironment.

### TECHNICAL SOLUTION

To accomplish the above object, according to one aspect of the present invention, there is provided
an artificial neural network training method comprising the steps of: generating a training data set including M pieces of individual training data (M is a natural number equal to or greater than 2), by a neural network training system; and training an artificial neural network on the basis of the training data set, by the neural network training system, wherein the step of generating a training data set including M pieces of individual training data includes the step of generating an m-th training data to be included in the training data set for all m where 1<=m<=M, wherein the step of generating an m-th training data includes the steps of: acquiring first to N-th pathology slide images (here, N is a natural number equal to or greater than 2), wherein the first to N-th pathology slide images are pathology slide images obtained by staining serial sections of a single pathological specimen with different staining reagents; and generating the m-th training data on the basis of the first to N-th pathology slide images.

In an embodiment, the step of generating the m-th training data on the basis of the first to N-th pathology slide images may include the step of converting the first to N-th pathology slide images into one multi-channel image through channel stacking, wherein the m-th training data includes the multi-channel image.

In an embodiment, the training data includes N channels, and the step of converting the first to N-th pathology slide images into one multi-channel image through channel stacking may include the step of constituting an n-th channel of the multi-channel image with each pixel value of the n-th pathology slide image for all natural numbers n where 1<=n<=N.

In an embodiment, the step of generating the m-th training data on the basis of the first to N-th pathology slide images may include the steps of: specifying a biological tissue area existing in each of the first to N-th pathology slide images; matching the first to N-th pathology slide images so that positions and shapes of the biological tissue areas existing in the first to N-th pathology slide images may match; and converting the matched first to N-th pathology slide images into one multi-channel image through channel stacking, wherein the m-th training data may include the multi-channel image.

In an embodiment, the training data includes N channels, and the step of converting the matched first to N-th pathology slide images into one multi-channel image through channel stacking may include the step of constituting an n-th channel of the multi-channel image with each pixel value of the matched n-th pathology slide image for all natural numbers n where 1<=n<=N.

In an embodiment, the step of matching the first to N-th pathology slide images so that the positions and shapes of the biological tissue areas existing in the first to N-th pathology slide images may match may include the step of calculating a conversion relation corresponding to an i-th pathology slide image for all natural numbers i where 1<=i<=N (here, the conversion relation corresponding to the i-th pathology slide image is a conversion relation between the i-th pathology slide image and a matched i-th pathology slide image corresponding thereto), and the step of generating the m-th training data on the basis of the first to N-th pathology slide images may further include the steps of: modifying a lesion annotation area assigned to a j-th pathology slide image using a conversion relation corresponding to the j-th pathology slide image; and converting the modified lesion annotation areas of the first to N-th pathology slide images into one multi-channel lesion annotation area through channel stacking, wherein the m-th training data further includes the multi-channel lesion annotation area.

According to another aspect of the present invention, there is provided a method of providing a result of determination on a predetermined determination target pathological specimen through an artificial neural network trained by the artificial neural network training method described above, the method comprising the steps of: acquiring first to N-th determination target pathology slide images (here, N is a natural number equal to or greater than 2), by a computing system, wherein the first to N-th determination target pathology slide images are pathology slide images in which serial sections of the determination target pathological specimen are stained with different staining reagents; and outputting a result of determination on the determination target pathological specimen determined by the artificial neural network on the basis of the first to N-th determination target pathology slide images, by the computing system.

According to another aspect of the present invention, there is provided a computer program installed in a data processing device and recorded on a medium for performing the method.

According to another aspect of the present invention, there is provided a computer-readable recording medium on which a computer program for performing the method described above is recorded.

According to another aspect of the present invention, there is provided an artificial neural network training system including a processor and a memory for storing a computer program, wherein the computer program, when executed by the processor, allows a computing system to perform a method of training an artificial neural network, and the artificial neural network training method of the artificial neural network training system comprises the steps of: generating a training data set including M pieces of individual training data (here, M is a natural number equal to or greater than 2), by the neural network training system; and training the artificial neural network on the basis of the training data set, by the neural network training system, wherein the step of generating a training data set including M pieces of individual training data includes the step of generating an m-th training data to be included in the training data set for all m where 1<=m<=M, wherein the step of generating an m-th training data includes the steps of: acquiring first to N-th pathology slide images (here, N is a natural number equal to or greater than 2), wherein the first to N-th pathology slide images are pathology slide images obtained by staining serial sections of a single pathological specimen with different staining reagents; and generating the m-th training data on the basis of the first to N-th pathology slide images.

There is provided an artificial neural network training system including a step of generating the m-th training data on the basis of the first to N-th pathology slide images.

According to another aspect of the present invention, there is provided a system for providing a result of determination on a pathological specimen, the system including a processor and a memory for storing a computer program, wherein the computer program, when executed by the processor, allows a computing system to perform a method of providing a result of determination on a pathological specimen through an artificial neural network trained by the artificial neural network training method described above, and the method of providing the determination result comprises the steps of: acquiring first to N-th determination target pathology slide images (here, N is a natural number equal to or greater than 2), by a computing system, wherein the first to N-th determination target pathology slide images are pathology slide images in which serial sections of a predetermined determination target pathological specimen are stained with different staining reagents; and outputting a result of determination on the determination target pathological specimen determined by the artificial neural network on the basis of the first to N-th determination target pathology slide images, by the computing system.

### ADVANTAGEOUS EFFECTS

According to the technical spirit of the present invention, it is possible to provide a method and system that can diagnose and prognose the current state of cancer, and grasp responsiveness to a specific treatment method with high accuracy by training an artificial neural network using pathology slides obtained by staining serial sections of a single pathological specimen with H&E or a variety of target IHC staining reagents so that the trained artificial neural network may comprehensively analyze the tumor microenvironment.

In addition, according to the technical spirit of the present invention, as it is allowed to use by stackin several sheets of pathology slide images generated by utilizing a widely used method, instead of a high-cost and non-generalized method such as multiplex IHC, an effect similar to the multiplex IHC can be obtained, and in addition, as the possibility of errors that may occur in the process of separating position information for each target can be blocked through color-filtering of multiplex IHC results, there is an effect of increasing accuracy of analyzing the tumor microenvironment.

### BRIEF DESCRIPTION OF THE DRAWINGS

In order to more fully understand the drawings cited in the detailed description of the present invention, a brief description of each drawing is provided.
FIG. 1 is a view schematically showing an environment in which a method of training an artificial neural network and a method of providing a result of determination on a pathological specimen according to the technical spirit of the present invention are performed.
FIG. 2 is a flowchart illustrating a neural network training method according to an embodiment of the present invention.
FIG. 3 is a view showing one multi-channel image generated by a plurality of pathology slide images expressed as an RGB color model through channel stacking.
FIG. 4 is a view showing an example of a process of generating individual training data according to an embodiment of the present invention.
FIG. 5 is a flowchart illustrating an example of a method of providing a result of determination on a pathological specimen according to an embodiment of the present invention.
FIG. 6 is a view showing a schematic configuration of an artificial neural network training system according to an embodiment of the present invention, and FIG. 7 is a view showing a schematic configuration of a determination result providing system according to an embodiment of the present invention.

### BEST MODE FOR CARRYING OUT THE INVENTION

Since the present invention may make various modifications and have various embodiments, specific embodiments will be illustrated in the drawings and described in detail in the detailed description. However, it should be understood that this is not intended to limit the present invention to the specific embodiments, and includes all modifications, equivalents, and substitutes included in the spirit and scope of the present invention. In describing the present invention, when it is determined that the detailed description of related known techniques may obscure the subject matters of the present invention, the detailed description will be omitted.

Terms such as first, second, and the like may be used to describe various components, but the components should not be limited by the terms. The terms such as first, second, and the like do not indicate a particular order and are used only for the purpose of distinguishing one component from the others.

Terms used in this application are only used to describe specific embodiments, and are not intended to limit the present invention. Singular expressions include plural expressions unless the context clearly dictates otherwise.

It should be understood that in this specification, terms such as "include", "have", and the like are intended to specify presence of a feature, a number, a step, an operation, a component, a part, or a combination thereof described in the specification, and do not preclude the possibility of presence or addition of one or more other features, numbers, steps, operations, components, parts, or combinations thereof.

In addition, when one component 'transmits' data to another component in this specification, it means that the component may directly transmit the data to another component, or may transmit the data to another component through at least one other component. Contrarily, when one component 'directly transmits' data to another component, it means that the data is transmitted from the component to another component without going through the other components.

Hereinafter, the present invention will be described in detail with reference to the accompanying drawings, focusing on the embodiments of the present invention. Like reference numerals in each figure indicate like elements.

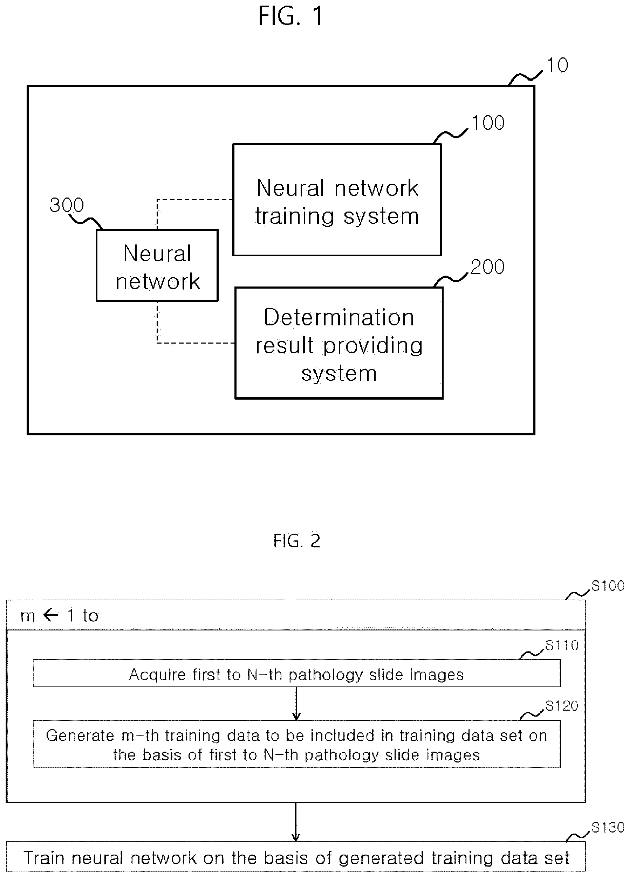

FIG. 1 is a view schematically showing an environment in which a method of training an artificial neural network and a method of providing a result of determination on a pathological specimen according to the technical spirit of the present invention are performed.

Referring to FIG. 1, an artificial neural network training method according to an embodiment of the present invention may be performed by a neural network training system 100, and a method of providing a result of determination on a pathological specimen according to an embodiment of the present invention may be performed by a system for providing a result of determination on a pathological specimen (hereinafter, referred to as a 'determination result providing system'). The neural network training system 100 may train an artificial neural network 300 for providing diagnosis information, prognosis information, and/or information on the response to a treatment method for a pathological specimen, and the determination result providing system 200 may make various determinations (e.g., expression and prognosis of a disease, determination of a treatment method, etc.) for a target specimen by using the trained artificial neural network 300.

The neural network training system 100 and/or the determination result providing system 200 may be a computing system, which is a data processing device having an arithmetic capability for implementing the technical spirit of the present invention, and generally, the systems may include a computing device such as a personal computer, a portable terminal, or the like, as well as a server, which is a data processing device that can be accessed by clients through a network.

Although the neural network training system 100 and/or the determination result providing system 200 may be implemented as any one physical device, those skilled in the art may easily infer that the neural network training system 100 and/or the determination result providing system 200 according to the technical spirit of the present invention may be implemented by organically combining a plurality of physical devices as needed.

The neural network training system 100 may train the neural network 300 on the basis of training data generated from a plurality of pathological specimens.

The pathological specimen may be a biological tissue sampled from various organs of a human body and resected through a biopsy or surgery. The neural network training system 100 may generate individual training data by using digital pathology slide images of serial sections of a pathological specimen, and train the neural network 300 by inputting the individual training data into the input layer of the neural network 300.

In an embodiment, the neural network 300 may be an artificial neural network trained to output a probability value of whether a disease is expressed for a predetermined disease. The neural network 300 may output a numerical value, i.e., a probability value, indicating a result of the determination on a target specimen (e.g., possibility of whether a disease is expressed) on the basis of the data input through an input layer.

In this specification, the artificial neural network is a neural network artificially constructed on the basis of the operating principles of human neurons, including a multi-layer perceptron model, and may mean a set of information expressing a series of designs defining the artificial neural network.

In an embodiment, the artificial neural network may be a convolutional neural network or may include a convolutional neural network.

Meanwhile, the trained neural network 300 may be stored in the determination result providing system 200, and the determination result providing system 200 may make a determination on a predetermined diagnosis target specimen using the trained artificial neural network.

As shown in FIG. 1, the neural network training system 100 and/or the determination result providing system 200 may be implemented in the form of a subsystem of a predetermined mother system 10. The server 10 means a data processing device having an arithmetic capability for implementing the technical spirit of the present invention, and those skilled in the art may easily infer that any device capable of performing a particular service, such as a personal computer, a portable terminal, or the like, as well as a data processing device that can be accessed by clients through a network, may be defined as a server.

Alternatively, according to embodiments, the neural network training system 100 and the determination result providing system 200 may be implemented in a form separated from each other.

FIG. 2 is a flowchart illustrating a neural network training method according to an embodiment of the present invention.

Referring to FIG. 2, the neural network training system 100 may generate a training data set including M pieces of individual training data (here, M is a natural number equal to or greater than 2). To this end, the neural network training system 100 may generate m-th training data to be included in the training data set for all m where 1<=m<=M (S100).

In order to generate the m-th training data to be included in the training data set, the neural network training system 100 may acquire first to N-th pathology slide images (here, N is a natural number equal to or greater than 2) (S110).

At this point, the first to N-th pathology slide images may be pathology slide images obtained by staining serial sections of a single pathological specimen with different staining reagents.

Each section of the pathological specimen may be a part of the pathological specimen sliced to manufacture a digital slide image, and the first to N-th pathology slide images may be generated by consecutively slicing the pathological specimen to manufacture a plurality of glass slides, staining the glass slides with different staining reagents, and digitizing the stained glass slides. At this point, the staining reagent may be a reagent for hematoxylin and eosin (H&E) staining or a reagent for immunohistochemistry (IHC) staining of a specific target.

For example, a plurality of pathology slide images corresponding to a pathological specimen may be generated by consecutively slicing the pathological specimen, sequentially staining each sliced section with an H&E staining reagent, a first IHC staining reagent, a second IHC staining reagent, or the like to manufacture glass slides, and digitally imaging the glass slides.

According to embodiments, the neural network training system 100 may receive the first to N-th pathology slide images corresponding to a predetermined pathological specimen from an external terminal, or may acquire the first to N-th pathology slide images from a memory device previously storing the first to N-th pathology slide images corresponding to the pathological specimen.

Meanwhile, the neural network training system 100 may generate the m-th training data on the basis of the first to N-th pathology slide images (S120).

In an embodiment, the neural network training system 100 may generate the m-th training data through a channel stacking method. That is, the neural network training system 100 may convert the first to N-th pathology slide images into one multi-channel image through channel stacking, and the m-th training data may include the multi-channel image.

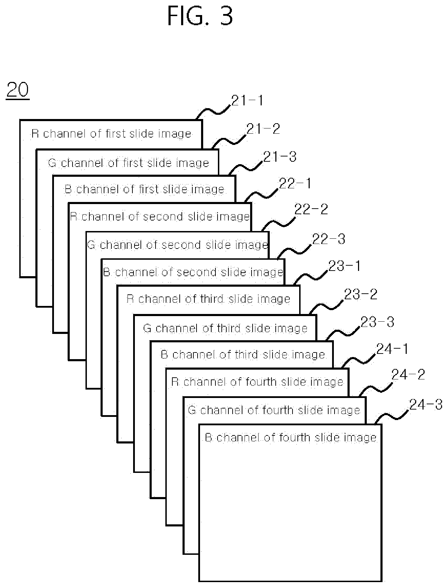

FIG. 3 is a view showing one multi-channel image generated by a plurality of pathology slide images expressed as an RGB color model through channel stacking. FIG. 3 shows a case in which slide images of four consecutive sections extracted from a single pathological specimen are converted into a multi-channel image.

Referring to FIG. 3, the multi-channel image 20 may be configured of 12 channels, which is a product of 4, i.e., the number of slide images, and 3, i.e., the number of channels constituting each slide image, and a first channel 21-1 may be configured of an R channel value of each pixel of a first slide image, a second channel 21-2 may be configured of a G channel value of each pixel of the first slide image, and a second channel 21-3 may be configured of a B channel value of each pixel of the first slide image, a fourth channel 22-1 may be configured of an R channel value of each pixel of a second slide image, a fifth channel 22-2 may be configured of a G channel value of each pixel of the second slide image, a sixth channel 22-3 may be configured of a B channel value of each pixel of the second slide image, a seventh channel 23-1 may be configured of an R channel value of each pixel of a third slide image, an eighth channel 23-2 may be configured of a G channel value of each pixel of the third slide image, a ninth channel 23-3 may be configured of a B channel value of each pixel of the third slide image, a tenth channel 24-1 may be configured of an R channel value of each pixel of a fourth slide image, a eleventh channel 24-2 may be configured of a G channel value of each pixel of the fourth slide image, and a twelfth channel 24-3 may be configured of a B channel value of each pixel of the fourth slide image.

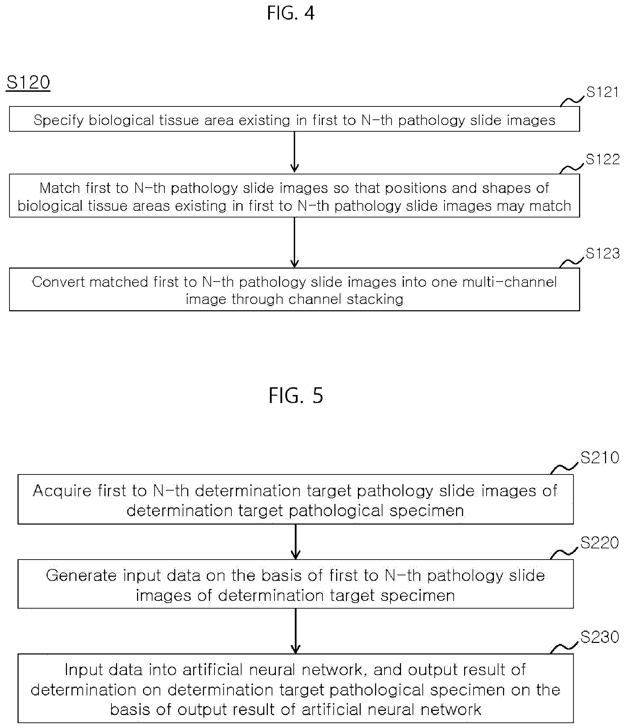

Meanwhile, each pathology slide image extracted from a single pathological specimen may be slightly misaligned in the position or direction during the process of manufacturing a corresponding slide image. In this case, a process of matching each pathology slide image should be performed before channel stacking is performed on each pathology slide image, and the flowchart of this case is shown in FIG. 4.

Referring to FIG. 4, the neural network training system 100 may specify a biological tissue area existing in each of the first to N-th pathology slide images (S121).

The neural network training system 100 may specify a biological tissue area from an image in various ways. In an embodiment, the neural network training system 100 may specify a biological tissue area using corresponding information when information on the biological tissue area is previously annotated in a slide image. Alternatively, the neural network training system 100 may specify the biological tissue area by using a previously trained neural network for determining a biological tissue area. Alternatively, the biological tissue area may be specified through various known methods.

Meanwhile, the neural network training system 100 may match the first to N-th pathology slide images so that the positions and shapes of the biological tissue areas existing in the first to N-th pathology slide images may match (S122). In an embodiment, for all natural numbers j where 2<=j<=N, the neural network training system 100 may repeatedly perform the process of matching the (j-1)-th pathology slide image and the j-th pathology slide image so that the positions and shapes of the biological tissue area in the (j-1)-th pathology slide image and the biological tissue area in the j-th pathology slide image may match.

The image matching is a technique used in the field, and means a processing technique of modifying images different from each other and displaying the images in a single coordinate system. The method of matching two images may include a method of converting the images so that the contours of the tissue areas included in the images are as similar as possible, a method of converting the images so that feature points within the tissue area match as much as possible, and the like, and specifically, a matching algorithm based on similarity between two images measured through Scale-Invariant Feature Transform (SIFT), Sum of Squared Difference (SSD), Sum of Absolute Difference (SAD), Normalized Cross Correlation (NCC), or the like may be used.

Meanwhile, a conversion relation between the matched pathology slides may be calculated in the process of matching the first to N-th pathology slide images. That is, the neural network training system 100 may calculate a conversion relation corresponding to the i-th pathology slide image for all natural numbers i where 1<=i<=N. Here, the conversion relation corresponding to the i-th pathology slide image is a conversion relation between the i-th pathology slide image and a matched i-th pathology slide image corresponding thereto.

Referring to FIG. 4 again, the neural network training system 100 may convert the matched first to N-th pathology slide images into one multi-channel image through channel stacking (S123), and since this is similar to those described above with reference to FIG. 3, detailed description thereof will be omitted.

In an embodiment, a lesion area may be previously annotated in each pathology slide image, and in this case, the previously annotated lesion area may be additionally included in the training data.

Describing this in more detail, for all natural numbers j where 1<=j<=N, the neural network training system 100 may modify the lesion annotation area assigned to the j-th pathology slide image using a conversion relation corresponding to the j-th pathology slide image, and convert the modified lesion annotation areas of the first to N-th pathology slide images into one multi-channel lesion annotation area through channel stacking, and the m-th training data may further include the multi-channel lesion annotation area.

On the other hand, when there is diagnosis information, prognosis information, and/or information on the response to a specific treatment method for a pathology specimen corresponding to the m-th training data, the neural network training system 100 may set the information as the label of the m-th training data.

When a training data set containing M pieces of individual training data is generated through the method described above, the neural network training system 100 may train the neural network 300 by inputting the generated training data set into the input layer of the neural network 300 (S130 in FIG. 2).

As it is allowed to use by stacking several sheets of pathology slide images generated by utilizing a widely used method, instead of a high-cost and non-generalized method such as multiplex IHC, the neural network training method according to the technical spirit of the present invention may obtain an effect similar to the multiplex IHC, and in addition, as the possibility of errors that may occur in the process of separating position information for each target can be blocked through color-filtering of multiplex IHC results, there is an effect of increasing accuracy of analyzing the tumor microenvironment.

FIG. 5 is a flowchart illustrating an example of a method of providing a result of determination on a pathological specimen according to an embodiment of the present invention. The method of providing a result of determination on a pathological specimen according to FIG. 5 may be performed by the determination result providing system 200, and the determination result providing system 200 may store the artificial neural network 300 trained by the neural network training system 100.

Referring to FIG. 5, the determination result providing system 200 may acquire first to N-th determination target pathology slide images of a predetermined determination target pathological specimen (S210). At this point, the first to N-th determination target pathology slide images are pathology slide images in which serial sections of the determination target pathological specimen are stained with different staining reagents.

The determination result providing system 200 may generate input data on the basis of the first to N-th pathology slide images of a determination target specimen (S220). Since the process of generating input data corresponding to the first to Nth pathology slide images of the determination target specimen is very similar to the process described above with reference to FIGS. 3 and 4, separate description thereof will be omitted.

The determination result providing system 200 may input the input data into the artificial neural network 300, and output a result of determination on the determination target pathological specimen on the basis of a result output from the artificial neural network (S230).

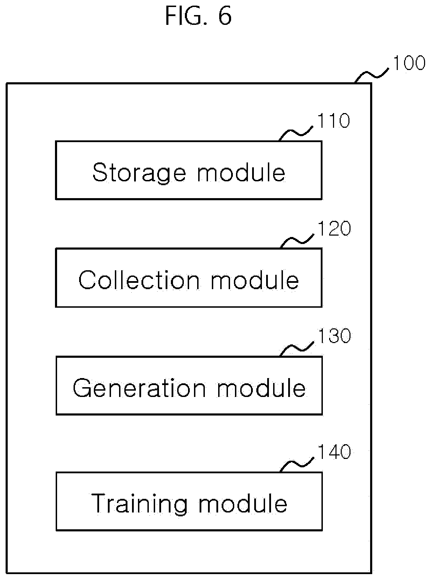

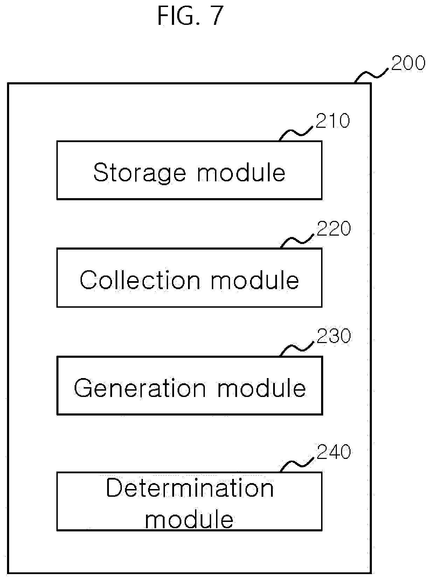

FIG. 6 is a view showing a schematic configuration of an artificial neural network training system 100 according to an embodiment of the present invention, and FIG. 7 is a view showing a schematic configuration of a determination result providing system 200 according to an embodiment of the present invention.

The artificial neural network training system 100 and the determination result providing system 200 may mean logical components having hardware resources and/or software required to implement the technical spirit of the present invention, and do not necessarily mean one physical component or a single device. That is, the artificial neural network training system 100 and the determination result providing system 200 may mean a logical combination of hardware and/or software provided to implement the technical spirit of the present invention, and may be implemented, when it is necessary, as a set of logical components for implementing the technical spirit of the present invention by being installed in the devices spaced apart from each other and performing respective functions. In addition, the artificial neural network training system 100 and the determination result providing system 200 may mean a set of components implemented separately for each function or role to implement the technical spirit of the present invention. The components of the artificial neural network training system 100 and the determination result providing system 200 may be placed in different physical devices or in the same physical device. In addition, according to implementation examples, combinations of software and/or hardware constituting each component of the artificial neural network training system 100 and the determination result providing system 200 may also be placed in different physical devices, and components placed in different physical devices may be organically combined with each other to implement each of the modules.

In addition, in this specification, a module may mean hardware for implementing the technical spirit of the present invention and a functional and structural combination of software for driving the hardware. For example, the module may mean a logical unit of a predetermined code and hardware resources for executing the predetermined code, and it can be easily inferred by those skilled in the art that the module does not necessarily mean physically connected codes or a kind of hardware.

Referring to FIG. 6, the artificial neural network training system 100 may include a storage module 110, an acquisition module 120, a generation module 130, and a training module 140. According to embodiments of the present invention, some of the components described above may not necessarily correspond to the components essential to implementation of the present invention, and in addition, according to embodiments, the artificial neural network training system 100 may include more components. For example, the artificial neural network training system 100 may further include a communication module (not shown) for communicating with external devices, and a control module (not shown) for controlling the components and resources of the artificial neural network training system 100.

The storage module 110 may store an artificial neural network 40 to be trained.

The acquisition module 120 may acquire first to N-th pathology slide images in which serial sections of each single pathological specimen are stained with different staining reagents.

The generation module 130 may generate individual training data on the basis of the first to N-th pathology slide images, and may configure a training data set including a plurality of individual training data.

The training module 140 may train the artificial neural network 300 on the basis of the training data set.

Referring to FIG. 7, the determination result providing system 200 may include a storage module 210, an acquisition module 220, a generation module 230, and a determination module 240. According to embodiments of the present invention, some of the components described above may not necessarily correspond to components essential to implementation of the present invention, and in addition, according to embodiments, the determination result providing system 200 may include more components. For example, the determination result providing system 200 may further include a communication module (not shown) for communicating with external devices, and a control module (not shown) for controlling the components and resources of the determination result providing system 200.

The storage module 210 may store the trained artificial neural network 40.

The acquisition module 220 may acquire first to N-th determination target pathology slide images in which serial sections of a predetermined determination target pathology specimen are stained with different staining reagents.

The generation module 230 may generate input data on the basis of the first to N-th determination target pathology slide images.

The determination module 240 may input the input data into the artificial neural network, and perform determination on the determination target specimen on the basis of a prediction value output from the artificial neural network 40.

Meanwhile, according to an implementation example, the artificial neural network training system 100 and the determination result providing system 200 may include a processor and a memory for storing programs executed by the processor. The processor may include a single-core CPU or a multi-core CPU. The memory may include high-speed random access memory and may also include one or more magnetic disk storage devices, flash memory devices, or other non-volatile memory devices such as non-volatile solid-state memory devices. Access to the memory by the processor and other components may be controlled by a memory controller.

Meanwhile, the method according to an embodiment of the present invention may be implemented in the form of computer-readable program instructions and stored in a computer-readable recording medium, and control programs and target programs according to an embodiment of the present invention may also be stored on the computer-readable recording medium. The computer-readable recording medium includes all types of recording devices in which data that can be read by a computer system is stored.

The program instructions recorded on the recording medium may be program instructions specially designed and configured for the present invention or known to and used by those skilled in the software field.

Examples of the computer-readable recording medium include magnetic media such as hard disks, floppy disks, and magnetic tapes, optical media such as CD-ROMs and DVDs, magnetooptical media such as floptical disks, and hardware devices specially configured to store and execute program instructions, such as ROM, RAM, flash memory, and the like. In addition, the computer-readable recording medium is distributed in computer systems connected through a network, so that computer-readable codes may be stored and executed in a distributed manner.

Examples of the program instructions include high-level language codes that can be executed by a device that electronically processes information using an interpreter or the like, e.g., a computer, as well as machine codes generated by a compiler.

The hardware device described above may be configured to operate as one or more software modules to perform the operations of the present invention, and vice versa.

The above description of the present invention is for illustrative purposes, and those skilled in the art may understand that it can be easily modified into other specific forms without changing the technical spirit or essential features of the present invention. Therefore, the embodiments described above should be understood as illustrative in all respects, not restrictive. For example, each component described as a single type may be implemented in a distributed manner, and similarly, components described as being distributed may be implemented in a combined form.

The scope of the present invention is indicated by the claims described below, rather than the detailed description described above, and all changes or modifications derived from the meaning and scope of the claims and equivalent concepts thereof should be construed as being included in the scope of the present invention.

### INDUSTRIAL APPLICABILITY

The present invention may be used in an artificial neural network training method for providing a result of determination on a pathological specimen, and a computing system for performing the same.

## Claims

1. An artificial neural network training method comprising the steps of:
generating a training data set including M pieces of individual training data (here, M is a natural number equal to or greater than 2), by a neural network training system; and
training an artificial neural network on the basis of the training data set, by the neural network training system, wherein
the step of generating a training data set including M pieces of individual training data includes the step of generating an m-th training data to be included in the training data set for all natural number m where 1<=m<=M, wherein
the step of generating an m-th training data includes the steps of:
acquiring first to N-th pathology slide images (here, N is a natural number equal to or greater than 2), wherein
the first to N-th pathology slide images are pathology slide images obtained by staining serial sections of a single pathological specimen with different staining reagents; and
generating the m-th training data on the basis of the first to N-th pathology slide images.

2. The method according to claim 1, wherein the step of generating the m-th training data on the basis of the first to N-th pathology slide images includes the step of converting the first to N-th pathology slide images into one multi-channel image through channel stacking, wherein
the m-th training data includes the multi-channel image.

3. The method according to claim 1, wherein the step of generating the m-th training data on the basis of the first to N-th pathology slide images includes the steps of:
specifying a biological tissue area existing in each of the first to N-th pathology slide images;
matching the first to N-th pathology slide images so that positions and shapes of the biological tissue areas existing in the first to N-th pathology slide images may match; and
converting the matched first to N-th pathology slide images into one multi-channel image through channel stacking, wherein
the m-th training data includes the multi-channel image.

4. The method according to claim 3, wherein the step of matching the first to N-th pathology slide images so that the positions and shapes of the biological tissue areas existing in the first to N-th pathology slide images may match includes the step of calculating a conversion relation corresponding to an i-th pathology slide image for all natural numbers i where 1<=i<=N (here, the conversion relation corresponding to the i-th pathology slide image is a conversion relation between the i-th pathology slide image and a matched i-th pathology slide image corresponding thereto), and
the step of generating the m-th training data on the basis of the first to N-th pathology slide images further includes the steps of:
modifying a lesion annotation area assigned to a j-th pathology slide image using a conversion relation corresponding to the j-th pathology slide image; and
converting the modified lesion annotation areas of the first to N-th pathology slide images into one multi-channel lesion annotation area through channel stacking, wherein
the m-th training data further includes the multi-channel lesion annotation area.

5. A method of providing a result of determination on a predetermined determination target pathological specimen through an artificial neural network trained by the artificial neural network training method described in claim 1, the method comprising the steps of:
acquiring first to N-th determination target pathology slide images (here, N is a natural number equal to or greater than 2), by a computing system, wherein the first to N-th determination target pathology slide images are pathology slide images in which serial sections of the determination target pathological specimen are stained with different staining reagents; and
outputting a result of determination on the determination target pathological specimen determined by the artificial neural network on the basis of the first to N-th determination target pathology slide images, by the computing system.

6. A computer program installed in a data processing device and recorded on a medium for performing the method according to any one of claims 1 to 5.

7. A computer-readable recording medium on which a computer program for performing the method according to any one of claims 1 to 5 is recorded.

8. An artificial neural network training system including a processor and a memory for storing a computer program, wherein the computer program, when executed by the processor, allows a computing system to perform a method of training an artificial neural network, and
the artificial neural network training method of the artificial neural network training system comprises the steps of:
generating a training data set including M pieces of individual training data (here, M is a natural number equal to or greater than 2), by the neural network training system; and
training the artificial neural network on the basis of the training data set, by the neural network training system, wherein
the step of generating a training data set including M pieces of individual training data includes the step of generating an m-th training data to be included in the training data set for all m where 1<=m<=M, wherein
the step of generating an m-th training data includes the steps of:
acquiring first to N-th pathology slide images (here, N is a natural number equal to or greater than 2), wherein the first to N-th pathology slide images are pathology slide images obtained by staining serial sections of a single pathological specimen with different staining reagents; and
generating the m-th training data on the basis of the first to N-th pathology slide images.

9. The system according to claim 8, wherein the step of generating the m-th training data on the basis of the first to N-th pathology slide images includes the step of converting the first to N-th pathology slide images into one multi-channel image through channel stacking, wherein the m-th training data includes the multi-channel image.

10. The system according to claim 8, wherein the step of generating the m-th training data on the basis of the first to N-th pathology slide images includes the steps of:
specifying a biological tissue area existing in each of the first to N-th pathology slide images;
matching the first to N-th pathology slide images so that positions and shapes of the biological tissue areas existing in the first to N-th pathology slide images may match; and
converting the matched first to N-th pathology slide images into one multi-channel image through channel stacking, wherein
the m-th training data includes the multi-channel image.

11. The system according to claim 10, wherein the step of matching the first to N-th pathology slide images so that the positions and shapes of the biological tissue areas existing in the first to N-th pathology slide images may match includes the step of calculating a conversion relation corresponding to an i-th pathology slide image for all natural numbers i where 1<=i<=N (here, the conversion relation corresponding to the i-th pathology slide image is a conversion relation between the i-th pathology slide image and a matched i-th pathology slide image corresponding thereto), and
the step of generating the m-th training data on the basis of the first to N-th pathology slide images includes the steps of:
modifying a lesion annotation area assigned to a j-th pathology slide image using a conversion relation corresponding to the j-th pathology slide image; and
converting the modified lesion annotation areas of the first to N-th pathology slide images into one multi-channel lesion annotation area through channel stacking, wherein
the m-th training data further includes the multi-channel lesion annotation area.

12. A system for providing a result of determination on a pathological specimen, the system including a processor and a memory for storing a computer program, wherein
the computer program, when executed by the processor, allows a computing system to perform a method of providing a result of determination on a pathological specimen through an artificial neural network trained by the artificial neural network training method described in claim 1, and
the method of providing the determination result comprises the steps of:
acquiring first to N-th determination target pathology slide images (here, N is a natural number equal to or greater than 2), by a computing system, wherein the first to N-th determination target pathology slide images are pathology slide images in which serial sections of a predetermined determination target pathological specimen are stained with different staining reagents; and
outputting a result of determination on the determination target pathological specimen determined by the artificial neural network on the basis of the first to N-th determination target pathology slide images, by the computing system.
